# EUROPÄISCHE PATENTSCHRIFT

(11) **EP 0 621 767 B1**
(45) Veröffentlichungstag und Bekanntmachung des Hinweises auf die Patenterteilung: **02.07.1997**
(21) Anmeldenummer: 94901810.5
(22) Anmeldetag: 16.11.1993
(51) Int. Cl.: A61F 2/40

(54) **SCHULTERGELENK-ENDOPROTHESE**
SHOULDER JOINT ENDOPROSTHESIS
ENDOPROTHESE DE L'ARTICULATION DE L'EPAULE

(30) Priorität: 17.11.1992 DE 4238832
(43) Veröffentlichungstag der Anmeldung: 02.11.1994
(73) Patentinhaber: Gerber, Bruno E., Dr., 2007 Neuchâtel (CH)
(72) Erfinder: CYPRIEN, Jean-Maxwell, CH-1562 Corcelles-près-Payerne (CH); GERBER, Bruno E., CH-2007 Neuchâtel (CH)
(74) Vertreter: Popp, Eugen, Dr.
(86) Internationale Anmeldenummer: EP9303218
(87) Internationale Veröffentlichungsnummer: WO9410941

(56) Entgegenhaltungen:
- EP-A- 0 299 889
- EP-A- 0 322 493
- EP-A- 0 329 854
- EP-A- 0 342 421
- WO-A-89/07917
- DE-A- 2 534 587
- DE-A- 2 714 387
- DE-A- 3 216 111
- US-A- 3 879 767
- US-A- 3 916 451
- US-A- 4 550 450

## Beschreibung

Die Erfindung betrifft eine Schultergelenk-Endoprothese.

In der Natur ist das Schultergelenk als ein Kugelgelenk ausgebildet, das einerseits eine flache Pfanne des Schulterblattes und andererseits einen großen, endseitigen Kopf des Oberarmknochens umfaßt, welche miteinander verbunden sind. Um das Schultergelenk ausgesprochen beweglich zu machen, ist die Gelenkkapsel weit ausgestaltet und sind die Bänder schwach ausgebildet. Auftretende Kräfte werden insofern in den Oberarmknochen über die Knochenrinden etc. abgeleitet, und zwar ohne einen sogenannten Kräftesprung. Die Halterung und Sicherung des Schultergelenkes erfolgt dabei ausschließlich durch Muskeln. So zieht sich beispielsweise selbst die Ursprungssehne des langen Bizepskopfes durch das Schultergelenk hindurch und verschiebt sich unter Druck gegen den Oberarmknochen.

Bei Zerstörung oder schwerer Veränderung des Schultergelenkes bzw. des Glenohumeral-Gelenkes, d. h. des Gelenkes zwischen Schulterblatt zum einen und Oberarmknochen zum anderen, sind in der Vergangenheit verschiedenste Vollgelenkersatzsysteme zur Implantation im Menschen vorgeschlagen worden. So ist beispielsweise in der DE 32 16 111 C2 eine Schultergelenk-Endoprothese beschrieben, die einen im Humerus zu verankernden Humerusschaft und einen in der Scapula zu verankernden Scapulaschaft aufweist, an deren freiem Ende jeweils ein Kugelkopf angebracht ist. Die beiden Kugelköpfe wiederum stehen über eine Art Kugelgelenk in Form eines kugelförmigen drehbaren Lagerteiles miteinander in Funktionseingriff. Ganz ähnlich verhält es sich mit der Schultergelenk-Endoprothese, die aus der DE 27 14 387 C3 bekannt ist. Als ausgesprochen nachteilig bei sämtlichen dieser Schultergelenk-Endoprothesen haben sich zum einen deren ausgesprochen große konstruktive Aufwand und zum anderen deren Kraftfluß herausgestellt. So werden insbesondere die Kräfte über die Kugel und dem jeweiligen Schaft unmittelbar auf das Schulterblatt bzw. auf den Oberarmknochen übertragen bzw. in das Schulterblatt oder in den Oberarmknochen unmittelbar eingeleitet. Dies hat einen enormen Kräftesprung und damit einhergehend einen Momentensprung zur Folge, woraus eine hohe Belastung des Schulterblattes bzw. des Oberarmknochens resultiert. Nicht selten kommt es daher zu einer Relativbewegung zwischen dem Schulterblatt und dem Scapulaschaft einerseits bzw. dem Oberarmknochen und dem Humerusschaft andererseits, welche wiederum eine nur geringe Haltbarkeit der Schultergelenk-Endoprothese insgesamt bzw. auch ein verlangsamtes Zusammenwachsen von Schulterblatt bzw. Oberarmknochen und Fremdkörper nach sich zieht. Die US-A-3 916 451 offenbart eine Schultergelenk-Endoprothese, umfassend ein glenoidales Gelenkteil, eine humerale Gelenkpfanne, die über einen intramedullären Prothesensteil am Oberarmknochen befestigbar ist, und eine Gelenkkugel, die zwischen dem glenoidalen Gelenkteil und der humeralen Gelenkpfanne beweglich angeordnet ist.

Der Erfindung liegt die Aufgabe zugrunde, eine Schultergelenk-Endoprothese bereitzustellen, die konstruktiv einfach ausgestaltet ist, einen besonders günstigen Kraftfluß bewirkt und eine hohe Knochenverträglichkeit aufweist.

Diese Aufgabe wird durch die erfindungsgemäße Schultergelenk-Endoprothese nach Anspruch 1 gelöst, die eine glenoidale Gelenkpfanne, welche dem Schulterblatt zugeordnet ist, eine humerale Gelenkpfanne, welche über einen intramedullären Prothesenstiel an dem Oberarmknochen befestigbar ist, und eine Gelenkkugel, welche von der glenoidalen Gelenkpfanne und der humeralen Gelenkpfanne nicht festgelegt und zwischen den Pfannen frei beweglich angeordnet ist, umfaßt.

Obschon die Ausgestaltung der Schultergelenk-Endoprothese nach der Erfindung eine Abkehr von der Natur in Folge der Dreiteiligkeit der Schultergelenk-Endoprothese, nämlich eine glenoidale Gelenkpfanne, eine humerale Gelenkpfanne und eine zwischen der glenoidalen Gelenkpfanne und der humeralen Gelenkpfanne frei beweglich angeordnete zentrale Gelenkkugel, bedeutet, lassen sich auf diese Weise zusätzliche Freiheitsgrade und damit eine ausgesprochen große Beweglichkeit erreichen. Dies hat eine sprungfreie Krafteinleitung in das Schulterblatt bzw. in den Oberarmknochen zur Folge. Scherbelastungen und Kippmomente auf die einzelnen Bestandteile der erfindungsgemäßen Schultergelenk-Endoprothese werden aufgrund der erfindungsgemäßen Anordnung stark reduziert, welche eine rollende als auch eine gleitende als auch eine translatorische Bewegung der Gelenkkugel zwischen der glenoidalen Gelenkpfanne und der humeralen Gelenkpfanne ermöglicht. Dies wiederum führt zu weit weniger Relativbewegungen zwischen dem Scapulaimplantat und dem Schulterblatt zum einen bzw. dem Humerusimplantat und dem Oberarmknochen zum anderen. Ein sogenannter Fremdkörpereffekt ist durch die erfindungsgemäße Schultergelenk-Endoprothese nicht ausgeschlossen, zumindest aber stark vermindert.

Zudem weist die Schultergelenk-Endoprothese nach der Erfindung eine besonders einfache Bauweise auf, da eine gegenseitige Festlegung der einzelnen Bestandteile, d. h. der glenoidalen Gelenkpfanne, der humeralen Gelenkpfanne und der zwischen der glenoidalen Gelenkpfanne und der humeralen Gelenkpfanne frei beweglich angeordneten Gelenkkugel, nicht erforderlich ist.

So wird die Schultergelenk-Endoprothese ähnlich dem natürlichen Schultergelenk nur durch Muskeln und Sehnen gehalten und gesichert. Dementsprechend wird die Gelenkkugel im hinteren Bereich des Schulterblattes von dem Muskulus Supra Spinatus und dem Muskulus Infra Spinatus stabilisiert sowie im vorderen Bereich durch den Muskulus Sub Scapularis gehalten. Schließlich wird die Gelenkkugel im oberen Bereich durch eine Sehnenplatte, nämlich durch die sogenannte Rotatorenmanschette zusammengehalten. Ein weiterer Zusammenhalt der erfindungsgemäßen Schultergelenk-Endoprothese ergibt sich durch sowohl die fibröse Gelenkkapsel als auch weitere Muskel bzw. Weichteile (sog. Hängematteneffekt). Schließlich baut sich in der bzw. um die Schultergelenk-Endoprothese - wie in der Natur auch - ein Vakuum auf, welches die glenoidale Gelenkpfanne, die humerale Gelenkpfanne und die zwischen diesen angeordnete Gelenkkugel sicher zusammenhält.

Von besonderem Vorteil bei der erfindungsgemäßen Schultergelenk-Endoprothese ist darüberhinaus die Möglichkeit der Verwendung bei einer nicht beschädigten, glenoidalen Gelenkpfanne eines Patienten, wie dies beispielsweise bei Humerusfrakturen mit Zerstörung des Kopfmassives oder bei aseptischer Nekrose vorkommen kann. Insoweit sind in diesem Fall von der erfindungsgemäßen Schultergelenk-Endoprothese lediglich die über einen intramedullären Prothesenstiel an dem Oberarmknochen befestigbare, humerale Gelenkpfanne einerseits und die Gelenkkugel andererseits zu implantieren, unter der Bedingung, daß keine begleitenden Lähmungen des Schultergürtels, des Deltamuskels oder der Rotatorenmanschettenmuskeln vorliegen.

Nach den Merkmalen des Anspruches 2, ist es ebensogut möglich, eine beispielsweise gänzlich zertrümmerte glenoidale Gelenkpfanne durch eine künstliche glenoidale Gelenkpfanne zu ersetzen, die über ein rückwärtiges Teil an dem Schulterblatt fixierbar ist.

Weiterhin liegt es im Rahmen der Erfindung nach den Ansprüchen 3 und 4, die glenoidale Gelenkpfanne aus Polyethylen oder dergleichen gleitlagerähnlichem Material zu bilden, das an dem rückwärtigen Teil der glenoidalen Gelenkpfanne angebracht ist, wobei der rückwärtige Teil aus Metall oder dergleichen, insbesondere aus Titan oder einer Titanlegierung, besteht. Auf diese Weise ist die erforderliche Verträglichkeit der gleonidalen Gelenkpfanne mit dem Knochenmaterial bzw. in diesem Fall dem Schulterblatt sichergestellt. Zudem wird durch die erfindungsgemäß Ausbildung der glenoidalen Gelenkpfanne aus Polyethylen oder dergleichen gleitlagerähnlichem Material eine zusätzliche Verminderung auftretender Scherkräfte und Kippmomente und somit ein schnelles Verwachsen des Knochenmaterials von dem Schulterblatt mit der glenoidalen Gelenkpfanne erreicht.

Weiterhin liegt es gemäß Anspruch 5 im Rahmen der Erfindung, das rückwärtige Teil oder dergleichen zum Verwachsen mit dem Knochen des Schulterblattes, insbesondere mit einem Porendurchmesser größer und/oder gleich 100 µm, auszubilden. Hierdurch wird die Haltbarkeit der glenoidalen Gelenkpfanne in dem Schulterblatt verbessert.

Von besonderem Interesse für eine weitere Veringerung von Scherkräften und Momenten, welche beim Patienten nicht selten zu merklichen Schmerzen fuhren, sind des weiteren die Merkmale des Anspruches 6, wonach die glenoidale Gelenkpfanne etwa schalen- und/oder in Draufsicht birnenförmig ausgebildet ist. Dabei weist vorzugsweise der obere Teil einen kleineren Öffnungsradius auf als der untere Teil. Durch eine solche, nicht sphäroide Ausgestaltung der glenoidalen Gelenkpfanne lassen sich zusätzliche Freiheitsgrade erhalten, die eine weitgehend sprungfreie Krafteinleitung in den Knochen ermöglichen.

Vorteilhafter Weise ist die glenoidale Gelenkpfanne darüberhinaus entsprechend Merkmal des Anspruches 7 mit einem Überhang oder dergleichen für einen neutrale ebenso wie für eine linke oder eine rechte glenoidale Gelenkpfanne versehen. Dies gestattet eine hohe Flexibilität bei der Implantation der glenoidalen Gelenkpfanne in Retroversion bezüglich der Schulterblattebene.

Die Merkmale nach den Ansprüchen 8 und 9, daß die humerale Gelenkpfanne aus Polyethylen oder dergleichen gleitlagerähnlichem Material gebildet ist, welches von dem Prothesenstiel aufgenommen ist, der selbst aus Metall oder dergleichen, insbesondere aus Titan oder einer Titanlegierung, besteht, sorgen für eine hohe Verträglichkeit zwischen der humeralen Gelenkpfanne mit dem Prothesenstiel zum einen und dem Knochenmaterial des Oberarmknochens zum anderen.

In vorteilhafter Weise ist der Prothesenstiel mit einem Plateauwinkel α zur Prothesenstielachse von ca. 50° intramedullär in dem Oberarmknochen fixierbar. Die gesamte humerale Gelenkpfanne läßt sich dabei in Retrotorsion bezüglich der Humeruskondylenebene implantieren.

In weiterer Ausgestaltung der Erfindung ist gemäß Anspruch 11 vorgesehen, daß die humerale Gelenkpfanne einen großen, vorzugsweise an die Gelenkkugel angepaßten, Innenradius aufweist. Somit ist eine hohe Beweglichkeit der Gelenkkugel zum Abrollen, Gleiten wie auch einer translatorischen Bewegung in bzw. an der humeralen Gelenkpfanne sichergestellt.

Von großer Bedeutung für eine zusätzliche Halterung und Sicherung der Gelenkkugel zwischen der glenoidalen Gelenkpfanne und der humeralen Gelenkpfanne in Richtung des unteren Körperendes ist die humerale Gelenkpfanne gemäß dem Merkmal des Anspruches 12 mit einem kaudalen bzw. nach dem unteren Körperende gerichteten Vorsprung oder dergleichen versehen. Ein solcher Vorsprung ermöglicht einerseits eine verbesserte Zentrierung der Gelenkkugel zwischen der glenoidalen Gelenkpfanne und der humeralen Gelenkpfanne im Falle eines seitlich ausgestreckten Armes und eine zusätzliche Abstützung der Gelenkkugel im Falle eines nach unten frei hängenden Armes.

Nach einem weiteren Merkmal entsprechend Anspruch 13 besteht die Gelenkkugel zur vorteilhaften Heraufsetzung der Verträglichkeit mit der insbesondere diese umgebenden fibrösen Gelenkkapsel aus Metall oder dergleichen. Zur Gewichtsreduzierung kann die Gelenkkugel dabei vorteilhafter Weise gemäß Anspruch 14 als Hohlkugel ausgebildet sein.

Durch die Maßnahme nach Anspruch 15, daß die Gelenkkugel in ihrer Größe abhängig von der jeweiligen Gelenkspannung und dem Innenvolumen der Rotatorenmanschette variierbar ist, wird ermöglicht, während der eigentlichen Operation durch Auswahl des Durchmessers der Gelenkkugel schnell, unkompliziert und individuell auf die von dem Operateur vorgefundenen Gegebenheiten im Schultergelenkbereich entsprechend flexibel zu reagieren. Demnach ist die Rekonstruktion und die Bestimmung des Innenvolumens in der Rotatorenmanschette unter gleichzeitiger Berücksichtigung der Gelenkspannung in einem Höchstmaß sichergestellt.

Schließlich ist die glenoidale Gelenkpfanne mit dem rückseitigen Teil über eine Rast-, Schnapp- oder dergleichen - vorrichtung entsprechend den Merkmalen nach Anspruch 16 verbindbar. Dies hat den Vorteil, daß einerseits die glenoidale Gelenkpfanne mit dem rückseitigen Teil ausgesprochen einfach verbunden werden kann und daß andererseits unterschiedlich geformte Gelenkpfannen ohne Aufwand unter individueller Anpassung an die jeweiligen Erfordernisse an einem jeweils als gleichgeformtes rückwärtiges Teil befestigt werden kann.

Weitere Merkmale, Vorteile und Einzelheiten der Erfindung ergeben sich aus der nachfolgenden Beschreibung einiger bevorzugter Ausführungsformen der Erfindung sowie anhand der Zeichnung. Hierbei zeigen:
- Fig. 1: eine schematische Seitenansicht einer Ausführungsform einer erfindungsgemäß ausgebildeten Schultergelenk-Endoprothese;
- Fig. 2: eine schematische Seitenansicht der glenoidalen Gelenkpfanne gemäß Fig. 1;
- Fig. 3: eine schematische Seitenansicht der humeralen Gelenkpfanne gemäß Fig. 1;
- Fig. 4: eine schematische Seitenansicht des intramedullären Prothesenstiels gemäß Fig. 1;
- Fig. 5A, 5B und 5C: schematische Seitenansichten der Gelenkkugel gemäß Fig. 1 sowie weiterer Gelenkkugeln mit größerem bzw. kleinerem Durchmesser;
- Fig. 6: eine schematische Seitenansicht einer anderen Ausführungsform einer erfindungsgemäßen Schultergelenk-Endoprothese mit einer Gelenkkugel entsprechend Fig. 5C;
- Fig. 7: eine schematische Seitenansicht einer Ausführungsform der glenoidalen Gelenkpfanne gemäß Fig. 1;
- Fig. 8: eine schematische Ansicht einer abgewandelten Ausführungsform der glenoidalen Gelenkpfanne gemäß Fig. 7;
- Fig. 9: eine Draufsicht auf die glenoidale Gelenkpfanne gemäß Pfeil 22 in Fig. 2 und 8;
- Fig. 10A, 10B und 10C: Querschnitte verschiedener Ausführungsformen von gleniodalen Gelenkpfannen gemäß Linie X - X in Fig. 9;
- Fig. 11: ein Längsschnitt durch die glenoidale Gelenkpfanne gemäß Linie XI - XI in Fig. 9;
- Fig. 12: ein schematischer Längsschnitt durch eine Ausführungsform des rückwärtigen Teiles;
- Fig. 13: ein schematischer Längsschnitt durch die Ausführungsform des rückwärtigen Teiles gemäß Fig. 12 mit Befestigungsschrauben;
- Fig. 14: einen schematischen Längsschnitt durch die Ausführungsform des rückwärtigen Teiles gemäß Fig. 13 mit einer damit verbundenen glenoidalen Gelenkpfanne entsprechend Fig. 7;
- Fig. 15: einen schematischen Längsschnitt durch die Ausführungsform des rückwärtigen Teiles gemäß Fig. 13 mit einer damit verbundenen glenoidalen Gelenkpfanne entsprechend Fig. 8;
- Fig. 16: eine schematische Seitenansicht der humeralen Gelenkpfanne gemäß Fig. 3 in vergrößerter Darstellung;
- Fig. 17: eine Draufsicht auf die humerale Gelenkpfanne gemäß Fig. 16 entsprechend Pfeil 64 in Fig. 16;
- Fig. 18: einen Querschnitt durch die humerale Gelenkpfanne längs der Linie XVIII - XVIII in Fig 17; und
- Fig. 19: einen Querschnitt durch die humerale Gelenkpfanne längs der Linie XIX - XIX in Fig. 17.

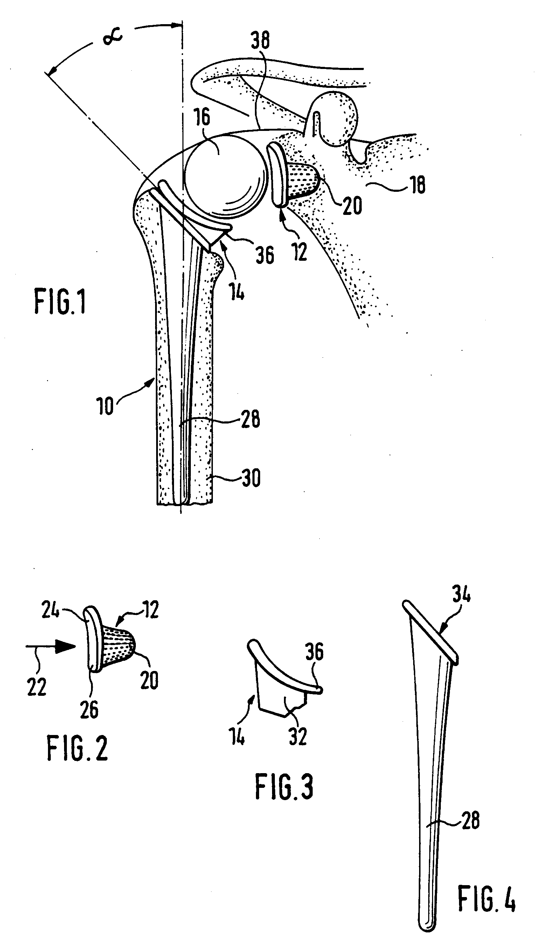

In Fig. 1 ist eine erste Ausführungsform einer Schultergelenk-Endoprothese 10 schematisch dargestellt. Die Schultergelenk-Endoprothese 10 umfaßt eine glenoidale Gelenkpfanne 12, eine humerale Gelenkpfanne 14 und eine zwischen der glenoidalen Gelenkpfanne 12 und der humeralen Gelenkpfanne 14 frei beweglich angeordnete Gelenkkugel 16.

Die glenoidale Gelenkpfanne 12 ist dem Schulterblatt 18 zugeordnet und an diesem über ein rückwertiges Teil 20 fixierbar. Die glenoidale Gelenkpfanne 12 besteht aus Polyethylen oder dergleichen gleitlagerähnlichem Material, das an dem rückwärtigen Teil 20 der glenoidalen Gelenkpfanne 12 angebracht bzw. von diesem aufgenommen ist. Das rückwärtige Teil 20 selbst ist aus Metall oder dergleichen, insbesondere Titan oder eine Titanlegierung, die mit dem Knochenmaterial des Schulterblattes 18 besonders verträglich ist.

Das rückwärtige Teil 20 ist - wie in Fig. 2 angedeutet - porös oder dergleichen zum Verwachsen mit dem Knochen des Schulterblattes ausgebildet. Insbesondere weist das rückwärtige Teil 20 eine Porenstruktur mit einem Porendurchmesser größer und/oder gleich 100 µm auf. Hierdurch werden das rückwärtige Teil 20 und damit zusammenhängend die von diesem aufgenommene glenoidale Gelenkpfanne 12 sicher in dem Knochen des Schulterblattes 18, der mit der Zeit in die einzelnen Poren des rückwärtigen Teiles 20 hineinwächst, gehalten.

Die glenoidale Gelenkpfanne 12 ist etwa schalenförmig bzw. in Draufsicht gemäß Pfeil 22 in Fig. 2 birnenartig ausgebildet. Dabei weist der obere Teil 24 der glenoidalen Gelenkpfanne 12 einen kleineren Öffnungsradius als der untere Teil 26 der glenoidalen Gelenkpfanne 12 auf. Zudem ist die glenoidale Gelenkpfanne 12 mit einem Überhang oder dergleichen versehen.

Die in Fig. 1 und 3 gezeigte humerale Gelenkpfanne 14 ist über einen intramedullären Prothesenstiel 28 an dem Oberarmknochen 30 befestigbar. Die humerale Gelenkpfanne 14 ist ebenfalls aus Polyethylen oder dergleichen gleitlagerähnlichem Material gebildet, das an dem Prothesenstiel 28 angebracht ist, beispielsweise über einen rückwärtigen Teil 32, der in eine Öffnung 34 des Prothesenstieles 28 einschiebbar und darin fixierbar ist. Der Prothesenstiel 28 selbst besteht aus Metall oder dergleichen, insbesondere aus Titan oder einer Titanlegierung, die besonders verträglich mit dem Prothesenstiel 28 umgebenden Knochenmaterial des Oberarmes 30 ist.

Wie in Fig. 1 angedeutet, ist der Prothesenstiel 28 mit einem Plateauwinkel α zur Prothesenstielachse von vorzugsweise ca. 50° bis 60° intramedullär fixiert.

Zur Erhöhung der Freiheitsgrade ist die humerale Gelenkpfanne 14 durch einen großen, vorzugsweise an die Gelenkkugel 16 angepaßten, Innenradius ausgestaltet. Weiterhin weist die humerale Gelenkpfanne 14 gemäß Fig. 1 und 3 einen Vorsprung 36 oder dergleichen auf, der sich kaudal bzw. nach dem unteren Körperende gerichtet erstreckt. Der Vorsprung 36 dient dabei in vorteilhafter Weise einerseits der Zentrierung der Gelenkkugel 16 bei seitlich ausgestrecktem Oberarmknochen 30, wobei die Gelenkkugel 16 gleichzeitig zum Beispiel durch den (nicht dargestellten) Muskulus Sub Scapularis gehalten ist, und andererseits der zusätzlichen Abstützung der Gelenkkugel 16 bei frei nach unten hängendem Oberarmknochen 30 entsprechend Fig. 1.

Die Gelenkkugel 16 selbst ist aus Metall oder dergleichen hergestellt, das mit der die Gelenkkugel 16 einschließenden fibrösen Gelenkkapsel verträglich ist. Vorzugsweise kann die Gelenkkugel 16 dabei als Hohlkugel ausgebildet sein.

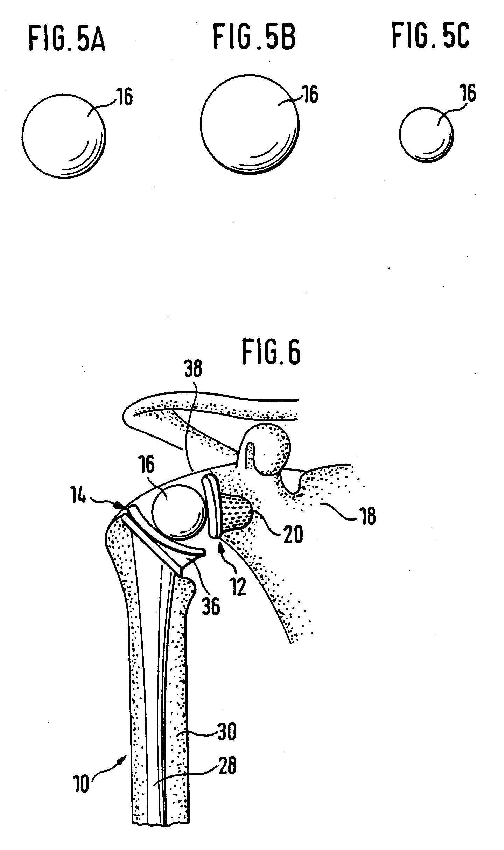

Entsprechend Fig. 5A bis 5C ist die Gelenkkugel 16 in ihrer Größe variierbar, und zwar jeweils abhängig von der entsprechenden Gelenkspannung und dem Innenvolumen der Rotatorenmanschette, welche von dem Operateur oftmals erst während des Eingriffes bestimmbar sind.

Durch die Ausgestaltung der erfindungsgemäßen Schultergelenk-Endoprothese 10 wird eine hohe Beweglichkeit mit mobilem Rotationszentrum garantiert und eine suffiziente Rekonstruktion der Rotationsmanschette unter Berücksichtigung einer adäquaten Gelenkspannung ermöglicht, und zwar bei gleichzeitiger Reduktion der Scherkräfte und Kippmomente auf die im Knochen des Schulterblattes 18 fixierte glenoidale Gelenkpfanne 12 bzw. auf die im Oberarmknochen 30 befestigte humerale Gelenkpfanne 14 und den humeralen Prothesenstiel 28.

Eine weitere Ausführungsform einer Erfindungsgemäßen Schultergelenk-Endoprothese 10 ist in Fig. 6 gezeigt. Diese zweite Ausführungsform der erfindungsgemäßen Schultergelenk-Endoprothese 10 nach Fig. 6 unterscheidet sich von derjenigen gemäß Fig. 1 lediglich durch eine Gelenkkugel 16 geringeren Durchmessers. Gleiche Teile sind insofern mit gleichen Bezugsziffern versehen.

Die Größe der Gelenkkugel 16 richtet sich beispielsweise nach dem Ausmaß der Beschädigung von der Rotatorenmanschette, welche mit der Bezugsziffer 38 angedeutet ist. Demnach besitzt die Gelenkkugel 16 einen kleinen Durchmesser entsprechend Fig. 6 im Falle eines großen Defektes der Rotatorenmanschette 38, wo hingegen die Gelenkkugel 16 einen großen Durchmesser entsprechend Fig. 1 im Falle eines kleinen Risses der Rotatorenmanschette 38 aufweist. Die übrigen eine Stabilisierung des Schultergelenkes wie auch eine Stabilisierung der erfindungsgemäßen Schultergelenk-Endoprothese 10 gemäß den Fig. 1 und 6 sicherstellenden Muskeln sind nicht näher dargestellt.

Die Erfindung ist dabei nicht auf die dargestellten Ausführungsformen beschränkt. Beispielsweise wäre es auch denkbar, sofern die Beschädigung der natürlichen glenoidalen Gelenkpfanne eines Patienten gering ist, lediglich eine humerale Gelenkpfanne 14 mit dazugehörigem intramedullären Prothesenstiel 28 an dem Oberarmknochen 30 zu befestigen und zwischen natürlicher glenoidaler Gelenkpfanne und implantierter humeraler Gelenkpfanne 28 eine entsprechend große Gelenkkugel 16 einzusetzen. Insbesondere bietet sich ein solches Vorgehen bei gewissen Humerusfraktorem mit Zerstörung des Kopfmassives oder aseptischer Nekrose an.

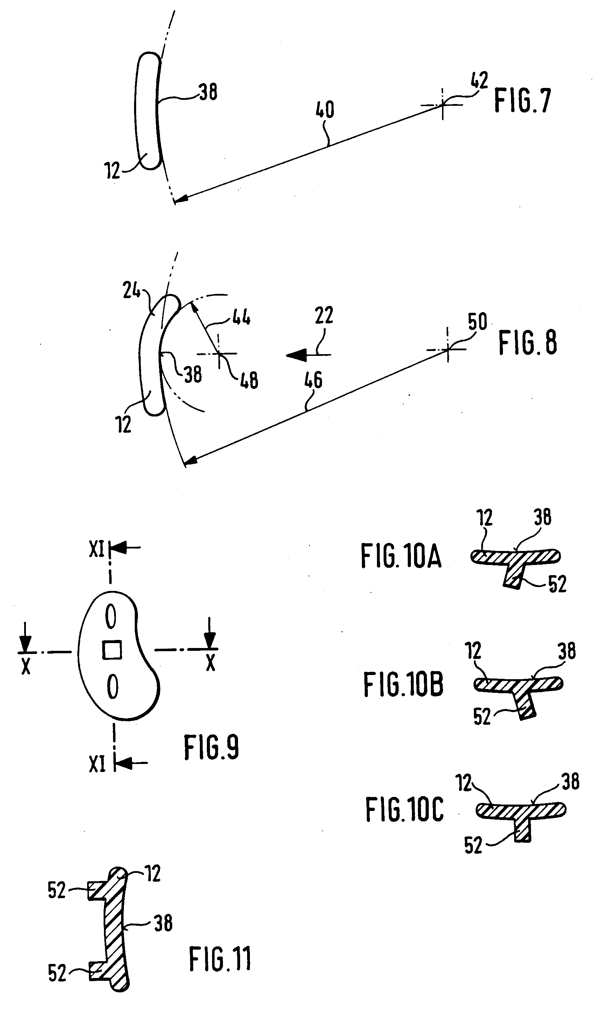

Bei der Ausführungsform der etwa schalenförmig ausgestalteten glenoidalen Gelenkpfanne 12 ist nach Fig. 7 die Innenfläche 38 der glenoidalen Gelenkpfanne 12 durch lediglich einen Öffnungsradius 40 mit dem Kreismittelpunkt 42 gebildet. Demgegenüber ist die Innenfläche 38 der glenoidalen Gelenkpfanne 12 nach Fig. 8 durch insgesamt 2 voneinander unterschiedlich großen Öffnungsradien 44, 46 mit deren Kreismittelpunkten 48, 50 gebildet. Entsprechend Fig. 8 ist der obere Teil 24 der glenoidalen Gelenkpfanne 12 mit dem kleineren Öffnungsradius 44, der untere Teil 26 der glenoidalen Gelenkpfanne 12 mit dem größeren Öffnungsradius 46 gebildet.

In den Fig. 9 bis 11 sind verschiedene Ausführungsformen der glenoidalen Gelenkpfanne 12 dargestellt, die gemäß Fig. 10A für eine linke Schulter und gemäß Fig. 10B für eine rechte Schulter verwendet werden kann. In Fig. 10C hingegen ist eine glenoidale Gelenkpfanne 12 als neutrales Element gezeigt. Die rückwärtigen Stifte 52 zum Befestigen der glenoidalen Gelenkpfanne 12 an dem rückwärtigen Teil 20 können dabei zur Erhöhung der Befestigungssicherheit perforiert sein (nicht dargestellt).

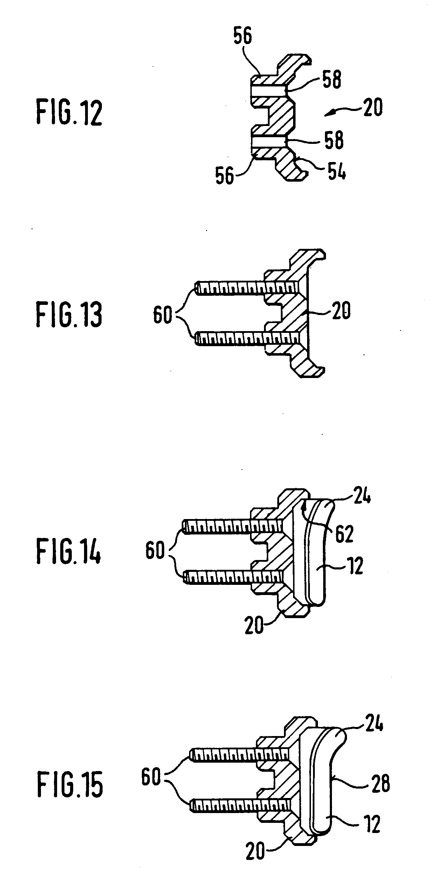

Das rückwärtige Teil 20 in Fig. 12 ist zur späteren Aufnahme einer glenoidalen Gelenkpfanne 12 an seiner vorderen Fläche 54 schalenförmig ausgebildet. Des weiteren sind an dem rückwärtigen Teil 20 zwei sich etwa von der vorderen Fläche 54 des rückwärtigen Teiles 20 senkrecht nach hinten wegerstreckende Vorsprünge 56 mit Bohrungen 58 angeformt. Das rückwärtige Teil 20 wird gemäß Fig. 13 über Schrauben 60 an dem jeweiligen Knochenmaterial befestigt, die sich jeweils durch die Bohrung 58 in dem entsprechenden Vorsprung 56 hindurcherstrecken.

Entsprechend den Fig. 14 und 15 kann nach erfolgter Befestigung des rückwärtigen Teiles 20 an dem Knochenmaterial durch Einbringung der Schrauben 60 die individuell ausgewählte glenoidale Gelenkpfanne 12 mittels einer Rast-, Schnapp- oder dergleichen -vorrichtung 62 auf die vordere Fläche 64 des rückwärtigen Teiles 20 aufgesetzt und von diesem sodann rastend bzw. schnappend aufgenommen werden. Die beiden Ausführungsformen nach Fig. 14 und 15 unterscheiden sich lediglich durch die unterschiedlich ausgestaltete Innenfläche 38 mit verschiedenen Öffnungsradien 40 bzw. 44, 46.

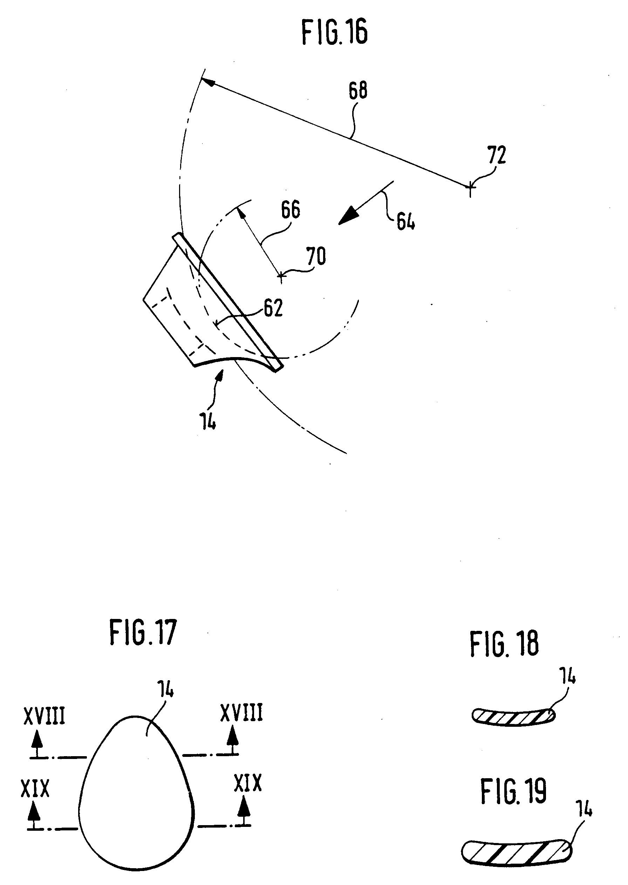

Die Ausführungsform der humeralen Gelenkpfanne 14 gemäß Fig. 16 besitzt ebenfalls eine Innenfläche 62 die schalenförmig bzw. in Draufsicht gemäß Pfeil 64 entsprechend Fig. 17 birnenartig ausgebildet ist. Die Innenfläche 62 ist ebenfalls aus zwei verschieden großen Öffnungsradien 66, 68 mit den jeweiligen Kreismittelpunkten 70, 72 zusammengesetzt. Wie deutlich aus Fig. 17 hervorgeht, weist die humerale Gelenkpfanne 14 eine ebenfalls etwa birnenartige Form auf.

## Patentansprüche

1. Schultergelenk-Endoprothese, umfassend eine glenoidale Gelenkpfanne (12), die dem Schulterblatt (18) zugeordnet ist, eine humerale Gelenkpfanne (14), die über einen intramedullären Prothesenstiel (28) an dem Oberarmknochen (30) befestigbar ist, und eine Gelenkkugel (16), die von der glenoidalen Gelenkpfanne (12) und der humeralen Gelenkpfanne (14) nicht festgelegt und zwischen den Pfannen (12, 14) frei beweglich angeordnet ist.

2. Schultergelenk-Endoprothese nach Anspruch 1,
dadurch gekennzeichnet,
daß die glenoidale Gelenkpfanne (12) über ein rückwärtiges Teil (20) an dem Schulterblatt (18) fixierbar ist.

3. Schultergelenk-Endoprothese nach Anspruch 2,
dadurch gekennzeichnet,
daß die glenoidale Gelenkpfanne (12) aus Polyethylen oder dergleichen gleitlagerähnlichem Material gebildet ist, das an dem rückwärtigen Teil (20) der glenoidalen Gelenkpfanne (12) angebracht ist.

4. Schultergelenk-Endoprothese nach Anspruch 2 und/oder 3,
dadurch gekennzeichnet,
daß das rückwärtige Teil (20) aus Metall oder dergleichen insbesondere aus Titan oder einer Titanlegierung, besteht.

5. Schultergelenk-Endoprothese nach Anspruch 2 bis 4,
dadurch gekennzeichnet,
daß das rückwärtige Teil (20) porös oder dergleichen zum Verwachsen mit dem Knochen des Schulterblattes (18), insbesondere mit einem Porendurchmesser größer und/oder gleich 100 µm, ausgebildet ist.

6. Schultergelenk-Endoprothese nach einem der Ansprüche 1 bis 5,
dadurch gekennzeichnet,
daß die glenoidale Gelenkpfanne (12) etwa schalen- und/oder in Draufsicht birnenförmig ausgebildet ist wobei der obere Teil (24) der glenoidalen Gelenkpfanne (12) einen kleineren Öffnungsradius aufweist als der untere Teil (26) der glenoidalen Gelenkpfanne (12).

7. Schultergelenk-Endoprothese nach einem der Ansprüche 1 bis 6,
dadurch gekennzeichnet,
daß die glenoidale Gelenkpfanne (12) einen Überhang oder dergleichen aufweist.

8. Schultergelenk-Endoprothese nach einem der Ansprüche 1 bis 7,
dadurch gekennzeichnet,
daß die humerale Gelenkpfanne (14) aus Polyethylen oder dergleichen gleitlagerähnlichem Material gebildet ist, das an dem Prothesenstiel (28) angebracht ist.

9. Schultergelenk-Endoprothese nach einem der Ansprüche 1 bis 8,
dadurch gekennzeichnet,
daß der Prothesenstiel (28) aus Metall oder dergleichen, insbesondere aus Titan oder einer Titanlegierung, besteht.

10. Schultergelenk-Endoprothese nach einem der Ansprüche 1 bis 9,
dadurch gekennzeichnet,
daß der Prothesenstiel (28) mit einem Plateauwinkel α zur Prothesenstielachse von ca. 50° bis 60° intramedullär fixierbar ist.

11. Schultergelenk-Endoprothese nach einem der Ansprüche 1 bis 10,
dadurch gekennzeichnet,
daß die humerale Gelenkpfanne (14) einen großen, vorzugsweise an die Gelenkkugel (16) angepaßten, Innenradius aufweist.

12. Schultergelenk-Endoprothese nach einem der Ansprüche 1 bis 11,
dadurch gekennzeichnet, daß die humerale Gelenkpfanne (14) einen kaudalen bzw. nach dem unteren Körperende gerichteten Vorsprung (36) oder dergleichen aufweist.

13. Schultergelenk-Endoprothese nach einem der Ansprüche 1 bis 12,
dadurch gekennzeichnet,
daß die Gelenkkugel (16) aus Metall oder dergleichen besteht.

14. Schultergelenk-Endoprothese nach einem der Ansprüche 1 bis 13,
dadurch gekennzeichnet,
daß die Gelenkkugel (16) als Hohlkugel ausgebildet ist.

15. Schultergelenk-Endoprothese nach einem der Ansprüche 1 bis 14,
dadurch gekennzeichnet,
daß die Gelenkkugel (16) in ihrer Größe abhängig von der jeweiligen Gelenkspannung und dem Innenvolumen der Rotatorenmanschette variierbar ist.

16. Schultergelenk-Endoprothese nach einem der Ansprüche 2 bis 15,
dadurch gekennzeichnet,
daß die glenoidale Gelenkpfanne (12) mit dem rückwärtigen Teil (20) über eine Rast-, Schnapp- oder dergleichen -Vorrichtung (62) verbindbar ist.

## Claims

1. Shoulder endoprosthesis, comprising a glenoid socket (12) which is assigned to the scapula (18, a humeral socket (14), which can be fastened to the humerus (30) via an intramedullary prosthesis stem (28), and a ball (16) which is not fixed by the glenoid socket (12) and the humeral socket (14) and is arranged so as to move freely between the sockets (12, 14).

2. Shoulder endoprosthesis according to Claim 1, characterized in that the glenoid socket (12) can be fixed to the scapula (18) via a backward part (20).

3. Shoulder endoprosthesis according to Claim 2, characterized in that the glenoid socket (12) is made of polyethylene or similar sliding-bearing material, which is applied to the backward part (20) of the glenoid socket (12).

4. Shoulder endoprosthesis according to Claim 2 and/or 3, characterized in that the backward part (20) is made of metal or the like, in particular of titanium or a titanium alloy.

5. Shoulder endoprosthesis according to one of Claims 2 to 4, characterized in that the backward part (20) is designed as porous or the like for incorporation with the bone of the scapula (18), in particular with a pore diameter of greater than and/or equal to 100 µm.

6. Shoulder endoprosthesis according to one of Claims 1 to 5, characterized in that the glenoid socket (12) is designed substantially in the shape of a shell and/or, in plan view, of a pear, the upper part (24) of the glenoid socket (12) having a smaller aperture radius than the lower part (26) of the glenoid socket (12).

7. Shoulder endoprosthesis according to one of Claims 1 to 6, characterized in that the glenoid socket (12) has an overhang or the like.

8. Shoulder endoprosthesis according to one of Claims 1 to 7, characterized in that the humeral socket (14) is made of polyethylene or similar sliding-bearing material, which is applied to the prosthesis stem (28).

9. Shoulder endoprosthesis according to one of Claims 1 to 8, characterized in that the prosthesis stem (28) is made of metal or the like, in particular of titanium or a titanium alloy.

10. Shoulder endoprosthesis according to one of Claims 1 to 9, characterized in that the prosthesis stem (28) is capable of intramedullary fixing with a plateau angle α with respect to the prosthesis stem axis of about 50° to 60°.

11. Shoulder endoprosthesis according to one of Claims 1 to 10, characterized in that the humeral socket (14) has a large internal radius, preferably matched to the ball (16).

12. Shoulder endoprosthesis according to one of Claims 1 to 11, characterized in that the humeral socket (14) has a caudal projection (36), or one directed towards the lower end of the body, or the like.

13. Shoulder endoprosthesis according to one of Claims 1 to 12, characterized in that the ball (16) is made of metal or the like.

14. Shoulder endoprosthesis according to one of Claims 1 to 13, characterized in that the ball (16) is designed as a hollow ball.

15. Shoulder endoprosthesis according to one of Claims 1 to 14, characterized in that the size of the ball (16) is variable as a function of the respective load on the joint and the internal volume of the rotator cuff.

16. Shoulder endoprosthesis according to one of Claims 2 to 15, characterized in that the glenoid socket (12) can be connected to the backward part (20) via a latch, snap-in or similar device (62).

## Revendications

1. Endoprothèse d'articulation de l'épaule comprenant une cavité glénoïdale d'articulation (12) qui est agencée sur l'omoplate (18), une cavité humérale d'articulation (14) fixée à l'humérus (30) au travers d'une hampe de prothèse (28) intramédullaire ainsi qu'une bille d'articulation (16) qui n'est fixée ni à la cavité glénoïdale d'articulation (12) ni à la cavité humérale d'articulation (14) et qui est mobile librement entre ces cavités (12,14).

2. Endoprothèse d'articulation de l'épaule selon la revendication 1, caractérisée en ce que la cavité glénoïdale d'articulation (12) peut être fixée à l'omoplate (18) par une pièce s'étendant vers l'arrière (20).

3. Endoprothèse d'articulation de l'épaule selon la revendication 2, caractérisée en ce que la cavité glénoïdale d'articulation (12) est réalisée en polyéthylène ou autre matériau analogue de coussinet qui est rapporté sur la pièce s'étendant vers l'arrière (20) de la cavité glénoïdale d'articulation (12).

4. Endoprothèse d'articulation de l'épaule selon la revendication 2 et/ou 3, caractérisée en ce que la pièce s'étendant vers l'arrière (20) est réalisée en métal ou analogue, en particulier en titane ou en alliage de titane.

5. Endoprothèse d'articulation de l'épaule selon les revendications 2 à 4, caractérisée en ce que la pièce s'étendant vers l'arrière (20) est réalisée de manière poreuse ou analogue pour s'entregreffer avec l'os de l'omoplate (18), en particulier avec un diamètre de porosité supérieur et/ou égal à 100 µm.

6. Endoprothèse d'articulation de l'épaule selon l'une des revendications 1 à 5, caractérisée en ce que la cavité glénoïdale d'articulation (12) est réalisée à la manière d'une coquille, et/ou en forme de poire telle que vue du dessus, forme dans laquelle la partie supérieure (24) de la cavité glénoïdale d'articulation (12) présente un rayon d'ouverture plus petit que celui de la partie inférieure (26) de cette cavité glénoïdale d'articulation (12).

7. Endoprothèse d'articulation de l'épaule selon l'une des revendications 1 à 6, caractérisée en ce que la cavité glénoïdale d'articulation (12) présente une excroissance ou analogue.

8. Endoprothèse d'articulation de l'épaule selon l'une des revendications 1 à 7, caractérisée en ce que la cavité humérale d'articulation (14) est réalisée en polyéthylène ou autre matériau analogue de coussinet qui est rapporté sur la hampe de prothèse (28).

9. Endoprothèse d'articulation de l'épaule selon l'une des revendications 1 à 8, caractérisée en ce que la hampe de prothèse (28) est réalisée en métal ou analogue, en particulier en titane ou en alliage de titane.

10. Endoprothèse d'articulation de l'épaule selon l'une des revendications 1 à 9, caractérisée en ce que la hampe de prothèse (28) peut être fixée de manière intramédullaire avec un angle de plateau a de 50° à 60° par rapport à l'axe de la hampe de prothèse.

11. Endoprothèse d'articulation de l'épaule selon l'une des revendications 1 à 10, caractérisée en ce que la cavité humérale d'articulation (14) présente un rayon interne important, de préférence correspondant à celui de la bille d'articulation (16).

12. Endoprothèse d'articulation de l'épaule selon l'une des revendications 1 à 11, caractérisée en ce que la cavité humérale d'articulation (14) présente une saillie caudale (36) ou analogue, c'est-à-dire orientée vers le bas de l'extrémité du corps.

13. Endoprothèse d'articulation de l'épaule selon l'une des revendications 1 à 12, caractérisée en ce que la bille d'articulation (16) est réalisée en métal ou analogue.

14. Endoprothèse d'articulation de l'épaule selon l'une des revendications 1 à 13, caractérisée en ce que la bille d'articulation (16) est réalisée sous la forme d'une bille creuse.

15. Endoprothèse d'articulation de l'épaule selon l'une des revendications 1 à 14, caractérisée en ce que la bille d'articulation (16) est variable dans ses dimensions en fonction des forces d'articulation respectives et du volume interne de la manchette de rotation.

16. Endoprothèse d'articulation de l'épaule selon l'une des revendications 2 à 15, caractérisée en ce que la cavité glénoïdale d'articulation (12) est fixée à la pièce s'étendant vers l'arrière (20) au moyen d'un dispositif (62) à encliquetage, à serrage élastique ou similaire.
